# EUROPÄISCHE PATENTSCHRIFT

(11) **EP 3 184 126 B1**
(45) Veröffentlichungstag und Bekanntmachung des Hinweises auf die Patenterteilung: **27.03.2019**
(21) Anmeldenummer: 16205591.7
(22) Anmeldetag: 21.12.2016
(51) Int. Cl.: A61L 27/16, A61L 27/50, A61L 27/54, A61L 24/00, A61L 24/06

(54) **ANTIBIOTISCHER POLYMETHYLMETHACRYLAT-KNOCHENZEMENT**
ANTIBIOTIC POLYMETHYL METHACRYLATE BONE CEMENT
CIMENT OSSEUX EN POLYMÉTHACRYLATE ANTIBIOTIQUE

(30) Priorität: 22.12.2015 DE 102015226501
(43) Veröffentlichungstag der Anmeldung: 28.06.2017
(73) Patentinhaber: Heraeus Medical GmbH, 61273 Wehrheim (DE)
(72) Erfinder: VOGT, Sebastian, 99092 Erfurt (DE); KLIMAS, Susann, 64295 Darmstadt (DE)
(74) Vertreter: Heraeus IP

(56) Entgegenhaltungen:
- EP-A1- 2 926 841
- DE-A1-102012 014 702
- S. S. RICHTER: "The in vitro activity of daptomycin against Staphylococcus aureus and Enterococcus species", JOURNAL OF ANTIMICROBIAL CHEMOTHERAPY, Bd. 52, Nr. 1, 12. Juni 2003 (2003-06-12), Seiten 123-127, XP55371268, DOI: 10.1093/jac/dkg288

## Beschreibung

Gegenstand der Erfindung ist ein antibiotischer Polymethylmethacrylat-Knochenzement, der Methylmethacrylat, mindestens ein Polymethylmethacrylat oder ein Polymethylmethacrylat-Copolymer, mindestens einen Polymerisationsinitiator, wie einen radikalischen Initiator, mindestens einen Polymerisationsbeschleuniger und mindestens einen Röntgenopaker enthält, wobei die Komponenten in einer pulverförmigen Komponente und einer pastenförmigen Komponente oder in zwei bei Raumtemperatur pastenförmigen Komponenten vorliegen. Der erfindungsgemäße Polymethylmethacrylat-Knochenzement enthält das cyclische Lipopeptid Daptomycin, ein pharmakologisch verträgliches Salz von Daptomycin, ein Solvat und/oder ein Hydrat enthaltend Daptomycin und mindestens zwei Calciumsalze mit unterschiedlicher Löslichkeit, die zwei unterschiedliche Freisetzungsprofile aufweisen.

Gegenstand der Erfindung ist ein antibiotischer Knochenzement, der für die Verankerung von Revisionsgelenkendoprothesen im Rahmen von einzeitigen und zweizeitigen septischen Revisionen vorgesehen ist, bei denen Gram-positive Bakterien, besonders Methicillin-resistente Staphylokokken (MRSA, MRSE) oder auch Vancomycin-resistente Staphylokocken für die zu Grunde liegende Infektion ursächlich sind. Daneben ist der antibiotische Polymethylmethacrylat-Knochenzement auch für die Herstellung von Spacern als temporäre Platzhalter bei zweizeitigen septischen Revisionen geeignet.

Gelenkendoprothesen werden in großem Umfang bei unterschiedlichsten Gelenkerkrankungen mit sehr großem Erfolg zur Erhaltung der Bewegungsfunktionen der Patienten eingesetzt. Leider kommt es bei einem geringen Anteil der Patienten zu Infektionen an den Gelenkendoprothesen und dem umgebenden Knochen- und Weichgewebe. Sehr häufig werden zur Behandlung dieser Infektionen der einzeitige und auch der zweizeitige Gelenkendoprothesenwechsel durchgeführt. Für die dauerhafte mechanische Fixierung der Revisions-Gelenkendoprothesen haben sich Revisions-Polymethylmethacrylat-Knochenzemente bewährt, die ein Antibiotikum oder zwei oder auch mehrere Antibiotika enthalten. Durch diese Antibiotika wird die Revisions-Gelenkendoprothese und das umgebende Knochen- und Weichgewebe zumindest unmittelbar postoperativ vor einer erneuten mikrobiellen Besiedlung geschützt. Neben der individuellen Beimischung von Antibiotika durch den Arzt haben sich industriell hergestellte Revisions-Polymethylmethacrylat-Knochenzemente bewährt. So werden von der Heraeus Medical GmbH die Revisions-Polymethylmethacrylat-Knochenzemente COPAL® G+C und COPAL® G+V hergestellt und vertrieben. COPAL® G+C enthält die Kombination Gentamicin und Clindamycin. COPAL® G+V enthält die Antibiotika Gentamicin und Vancomycin. Die
Kombination Gentamicin mit Vancomycin eignet sich bisher besonders dann, wenn die Infektion der Gelenkendoprothese durch Methicillin-resistente Staphylokokken (MRSA, MRSE) hervorgerufen wird. Seit einigen Jahren sind jedoch auch Vancomycin-resistente Staphylokokken- und Enterokokken-Stämme bekannt. Es ist damit zu rechnen, dass diese Vancomycin-resistenten Bakterien in den nächsten Jahren, neben den bisherigen MRSA/MRSE eine zunehmende Rolle als Verursacher von Gelenk-assoziierten Infektionen spielen werden. Daher ist es sinnvoll, einen Revisions-Polymethylmethacrylat-Knochenzement zu entwickeln, der mindestens ein Antibiotikum enthält, das wirksam gegenüber Vancomycin-resistenten Bakterien ist.

Implantat-assoziierte Infektionen mit multiresistenten Gram-positiven Bakterien, insbesondere mit Vancomycin-resistenten Bakterien, sind extrem schwierig zu behandeln und stellen eine ernste Gefahr für die Patienten dar. Deshalb sind für solche schwierigen Implantat-assoziierten Infektionen Revisionszemente wünschenswert, die solche Antibiotika enthalten, die einen sicheren lokalen Schutz vor Reinfektionen durch diese Problemkeime erwarten lassen.

Ein gegenüber Vancomycin-resistenten Bakterien wirksames Antibiotikum stellt Daptomycin (CAS 103060-53-3) dar. Daptomycin ist ein cyclisches Lipopeptid. Der Wirkungsmechanismus unterscheidet sich deutlich von dem der Glykopeptid-Antibiotika, wie Vancomycin und Teicoplanin. Daher ist Daptomycin im Allgemeinen auch bei Vancomycin-resistenten Gram-positiven Bakterien antimikrobiell wirksam. Daptomycin lagert sich in die Zellmembranen von Gram-positiven Bakterien ein und bildet Poren, durch die Kalium-Ionen aus dem Cytoplasma der Bakterienzellen in die Umgebung austreten können. Durch den Ausstrom von Kalium-Ionen depolarisieren die Bakterienzellen und sterben ab. Für die Porenbildung benötigt Daptomycin Calciumionen als Co-Faktor (G. M. Eliopoules, S. Wiley, E. Reiszner, P. G. Spitzer, G. Caputo, R. C. Moellering: In vitro and in vivo activity of LY146032, a new lipopeptide antibiotic. Antimicrob. Agents Chemother. 30 (1986) 532-535.; R. N. Jones, A. L. Barry: Antimicrobial activity and spectrum of LY146032, a lipopeptide antibiotic, including susceptibility testing recommendations. Antimicrob. Agents Chemother. 31 (1987) 625-629.).

Die bisher in Polymethylmethacrylat-Knochenzementen verwendeten Antibiotika, wie Gentamicin, Tobramycin, Vancomycin, Clindamycin, Erythromycin und Colistin erfordern keine Co-Faktoren, um antimikrobiell wirksam zu sein. Somit war bei den bisher bekannten mit Antibiotika-modifizierten Polymethylmethacrylat-Knochenzementen eine lokale antimikrobielle Wirkung beim Herauslösen der Antibiotika aus den ausgehärteten Polymethylmethacrylat-Knochenzementen durch Körperflüssigkeiten, wie Wundexsudat und Blut, gegeben, ohne dass Bestandteile der Körperflüssigkeiten als Co-Faktoren für die notwendig gewesen sind.
Bei einzeitigen und auch zweizeitigen septischen Revisionen wird das infizierte Knochen- und Weichgewebe nach Explantation der infizierten Gelenkendoprothese radikal debridiert, d.h. chirurgisch abgetragen. Sowohl bei einzeitigen als auch bei zweizeitigen Revisionen wird deshalb eine Bildung von Wundexsudat beobachtet. Das Wundexsudat dient zum Abtransport von Debris, wie Zelltrümmern und anderen Geweberesten. Es bildet sich an der Grenzfläche zwischen dem zur mechanischen Fixierung der Revisionsendoprothese benutzten Knochenzement und der Oberfläche des zuvor debridierten knöchernen Implantatlagers beim einzeitigen Wechsel.
Bei zweizeitigen Revisionen bildet sich einmal nach dem ersten Debridement Wundexsudat zwischen der Spaceroberfläche und dem Knochen- bzw. Weichgewebe und dann noch einmal nach dem zweiten Debridement bei der Implantation der
Revisionsgelenkendoprothese. Das Wundexsudat wird üblicherweise durch Drainagen aus dem Patienten geleitet. Die Drainagen verbleiben im Patienten bis der Wundexsudat-Strom nachgelassen hat. Die Wundexsudat-Menge kann dabei zwischen wenigen hundert Millilitern bis zu mehreren Litern Patienten-individuell variieren. Wundexsudat besteht hauptsächlich aus Wasser und Proteinen. Es enthält keine definierten Konzentrationen an Alkali- und Erdalkali-Ionen. Die Zusammensetzung variiert sehr stark. Durch das Wundexsudat kommt es zur Herauslösung von Antibiotika aus der Oberfläche von antibiotisch modifizierten Polymethylmethacrylat-Knochenzementen. Bei der Verwendung von Antibiotika, die keine Co-Faktoren für ihre antimikrobielle Wirksamkeit benötigen, ist dadurch immer ein antibiotischer Schutz der Zementoberfläche und des umgebenden Knochengewebes durch die im Wundsekret gelösten Antibiotika gegeben. Bei Daptomycin hängt dagegen die Wirksamkeit von der Anwesenheit einer hinreichend hohen Calciumionenkonzentration ab. Diese kann jedoch Patienten-individuell schwanken. Eine reproduzierbare lokale antimikrobielle Wirksamkeit eines nur mit Daptomycin ausgerüsteten Polymethylmethacrylat-Knochenzementes ist dadurch nicht gegeben.

DE 10 2012 014702 A offenbart einen pastenförmigen Knochenzement. In einem Ausführungsbeispiel wird eine Paste enthaltend Methylmethacrylat und Poly(methylmethacrylat-co-methylmethacrylat mit einem Zusatz von Daptomycin und eine Paste enthaltend Cumolhydroperoxid, Calciumcarbonat und Zirkoniumdioxid als Röntgenopaker) vermischt.

Aufgabe der Erfindung ist es, einen antimikrobiell wirksamen Polymethylmethacrylat-Knochenzement zu entwickeln, der das Antibiotikum Daptomycin enthält und der eine garantierte lokale antimikrobielle Wirksamkeit des Daptomycins ermöglicht, wenn das Daptomycin durch wässrige Körperflüssigkeiten, wie Wundexsudat und Blut, aus der Oberfläche des ausgehärteten Zementes herausgelöst wird, unabhängig von der chemischen Zusammensetzung der umgebenden wässrigen Körperflüssigkeiten.
Die Erfindung beruht auf dem überraschenden Befund, dass eine synchrone Freisetzung von Daptomycin und Calciumionen aus einem Polymethylmethacrylat-Knochenzement über einen Zeitraum von mehreren Tagen möglich ist, so dass eine lokale antimikrobielle Wirksamkeit des Daptomycins auch in Gegenwart von Körperflüssigkeiten erfolgt, in denen keine ausreichenden Mengen an Calciumionen vorhanden sind. Dadurch ist es möglich, einen optimalen lokalen antimikrobiellen Schutz der Zementoberfläche, der Oberfläche von Revisions-Gelenkendoprothesen und des den Revisionszement umgebenden Knochengewebes zu erreichen, auch wenn große Mengen an Wundexsudat mit niedrigen bis sehr niedrigen Calciumionen-Gehalten vom knöchernen Implantatlager und dem umgebenden Weichgewebe freigesetzt werden. Die Erfindung basiert darauf, ein in Wasser leicht lösliches, physiologisch unbedenkliches Calciumsalz und ein in Wasser gering lösliches, physiologisch unbedenkliches Calciumsalz zusammen mit Daptomycin in den Polymethylmethacrylat-Knochenzement zu integrieren. Das leicht lösliche Calciumsalz bewirkt eine initial hohe Freisetzung von Calciumionen, parallel zur initial hohen Freisetzung von Daptomycin. Anschließend werden geringe Mengen Daptomycin über mehrere Tage abgegeben. Dazu parallel werden geringe Mengen Calciumionen verzögert aus dem gering löslichen Calciumsalz freigesetzt. Dadurch ist sowohl die initiale antimikrobielle Wirkung des Daptomycins über die ersten zwei Tage als auch die antimikrobielle Wirkung über die nachfolgenden Tage gewährleistet.

Gelöst wurde die Aufgabe mit dem Knochenzement nach Anspruch 1, mit einem lokal freisetzenden Wirkstoffträger nach Anspruch 13, sowie mit einem Formkörper zur Anwendung in einem Verfahren zur Behandlung und/oder Prävention von bakteriellen Infektionen nach Anspruch 15. Bevorzugte Ausführungsformen sind in den Unteransprüchen sowie in der Beschreibung detailliert dargelegt.
Gegenstand der Erfindung ist ein antibiotischer, polymerisierbarer Knochenzement gemäß Anspruch 1.

Optional weist mindestens eine der Komponenten 1, 2 und/oder 3 eine mittlere Partikelgröße von 1 bis 250 µm, bestimmt mittels Sedimentationsanalyse, auf.

Ferner ist Gegenstand der Erfindung ein Knochenzement-Kit, enthaltend zwei Komponenten A und B, wobei
(i) Komponente A als Paste vorliegt und enthält
   (a1) mindestens ein radikalisch polymerisierbares Monomer,
   (a2) mindestens ein organisches Polymer umfassend mindestens ein Polymethylmethacrylat und/oder ein Polymethylmethacrylat-Copolymer, und
   (a3) mindestens einen Polymerisationsinitiator; und
   Komponente B als Paste vorliegt und umfasst
   (b1) mindestens ein radikalisch polymerisierbares Monomer,
   (b2) mindestens ein organisches Polymer umfassend mindestens ein Polymethylmethacrylat und/oder ein Polymethylmethacrylat-Copolymer, und
   (b3) mindestens einen Polymerisationsbeschleuniger; oder
(ii) Komponente A als Pulver vorliegt und umfasst
   (a1) mindestens ein pulverförmiges Polymer umfassend mindestens ein Polymethylmethacrylat und/oder ein pulverförmiges Gemisch umfassend Polymethylmethacrylat-Co-Polymere,
   (a2) mindestens einen pulverförmigen Röntgenopaker, und
   (a3) mindestens einen Polymerisationsinitiator; und
   Komponente B als Flüssigkeit oder Paste vorliegt und umfasst
   (b1) mindestens ein radikalisch polymerisierbares Monomer,
   (b2) optional mindestens ein organisches Polymer umfassend mindestens ein Polymethylmethacrylat und/oder ein Polymethylmethacrylat-Copolymer, und
   (b3) mindestens einen Polymerisationsbeschleuniger; wobei das Kit ferner umfasst als Komponente **1** Daptomycin, ein pharmakologisch verträgliches Salz von Daptomycin, eine polymorphe Form des Daptomycins, ein Solvat und/oder ein Hydrat enthaltend Daptomycin, und
   eine Kombination enthaltend
   a) als Komponente 2 ein in Wasser lösliches Calciumsalz mit einer Löslichkeit in Wasser bei Raumtemperatur von größer gleich 5 g/l, und
(iii) mindestens einen Polymerisationsinitiator,
(iv) mindestens einen Röntgenopaker,
(v) als Komponente **1** Daptomycin, ein pharmakologisch verträgliches Salz von Daptomycin, eine polymorphe Form des Daptomycins, ein Solvat und/oder ein Hydrat enthaltend Daptomycin, dadurch gekennzeichnet, dass
   der Knochenzement ferner umfasst
(vi) eine Kombination enthaltend
   a) als Komponente 2 ein in Wasser lösliches Calciumsalz mit einer Löslichkeit in Wasser bei Raumtemperatur von größer gleich 5 g/l, und
   b) als Komponente 3 ein in Wasser gering lösliches Calciumsalz mit einer Löslichkeit in Wasser bei Raumtemperatur von kleiner 5 g/l.

Erfindungsgemäß enthält der Knochenzement ein schnellfreisetzendes Calciumsalz und ein retardiert freisetzendes Calciumsalz. Vorzugsweise liegt das mindestens eine Calciumsalz mit einer mittleren Partikelgröße von 1 bis 250 µm vor. Nach alternativen Ausführungsformen liegt das mindestens eine Calciumsalz mit mindestens zwei verschiedenen mittleren Partikelgrößen vor, über die die Freisetzung definiert wird. So kann eine Fraktion eine mittlere Partikelgröße von 10 bis 150 µm und die mindestens zweite Fraktion eine mittlere Partikelgröße von 160 bis 250 µm aufweisen. Sämtliche in diesem Dokument angegebenen mittleren Partikelgrößen werden mittels Sedimentationsanalyse bestimmt.

Ferner ist es bevorzugt, wenn die Komponente **1** Daptomycin, ein pharmakologisch verträgliches Salz von Daptomycin, ein Solvat und/oder ein Hydrat enthaltend Daptomycin in einer Partikelgröße von 1 bis 250 µm ist, insbesondere mit einer mittleren Partikelgröße von 10 µm bis 250 µm, bevorzugt 100 bis 250 µm.

Das Freisetzungsprofil des mindestens einen Calciumsalzes kann auf unterschiedlichste Art und Weise gesteuert werden. So kann das Freisetzungsprofil gesteuert werden, indem Calciumsalze mit unterschiedlicher Löslichkeit in dem Knochenzement eingesetzt werden.

Erfindungsgemäß enthält der Knochenzement als (vi) eine Kombination enthaltend mindestens zwei Calciumsalze mit unterschiedlicher Löslichkeit in Wasser, nämlich
a) als Komponente **2** ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l,
   wobei mindestens die Komponente A als
   (a4) mindestens eine der Komponenten **1, 2** und **3** umfasst, die ausgewählt ist aus der Komponente **1,** Daptomycin, einem pharmakologisch verträglichen Salz von Daptomycin, einem Solvat und/oder einem Hydrat enthaltend Daptomycin, der Komponente **2,** einem in Wasser löslichem Calciumsalz mit einer Löslichkeit in Wasser bei Raumtemperatur von größer gleich 5 g/l, und der Komponente **3,** einem in Wasser gering löslichem Calciumsalz mit einer Löslichkeit in Wasser bei Raumtemperatur von kleiner 5 g/l, und/oder
      wobei die Komponente B als Paste vorliegt und als
   (b4) mindestens eine der Komponenten **1, 2** und **3** umfasst, die ausgewählt ist aus der Komponente **1,** Daptomycin, einem pharmakologisch verträglichen Salz von Daptomycin, einem Solvat und/oder einem Hydrat enthaltend Daptomycin, der Komponente **2,** einem in Wasser löslichem Calciumsalz mit einer Löslichkeit in Wasser bei Raumtemperatur von größer gleich 5 g/l, und der Komponente **3,** einem in Wasser gering löslichem Calciumsalz mit einer Löslichkeit in Wasser bei Raumtemperatur von kleiner 5 g/l.

Vorzugsweise weist die Komponente A und/oder B jeweils unabhängig mindestens die Komponenten **1** und **2,** die Komponenten **1** und **3** oder die Komponenten **1, 2** und **3** auf. Alternativ weist die Komponente A mindestens die Komponenten **1** und optional **2** oder die Komponenten **1** und optional **3** oder die Komponenten **1, 2** und **3** auf, wobei gleichzeitig die Komponente B mindestens die Komponenten **2** und optional **1** oder die Komponenten **3** und optional **1** oder die Komponenten **1, 2** und **3** aufweist. Nach einer weiteren Alternative weist die Komponente B mindestens die Komponenten **1** und optional **2** oder die Komponenten **1** und optional **3** oder die Komponenten **1, 2** und **3** auf, wobei gleichzeitig die Komponente A mindestens die Komponenten **2** und optional **1** oder die Komponenten **3** und optional **1** oder die Komponeten **1, 2** und **3** aufweist.

Die Komponenten **1, 2** und **3** sind vorzugsweise als separate Partikel im Polymethylmethacrylat-Knochenzement enthalten, wobei diese Partikel bevorzugt in der Pulverkomponente oder in mindestens einer pastenförmigen Komponente des Polymethylmethacrylat-Knochenzementes enthalten sind.

In einer vorteilhaften Ausgestaltung der Erfindung sind die Partikel der Komponenten **1, 2** und **3** jeweils als Kombination in einer partikuläre Formulierung enthalten, wobei diese partikuläre Formulierung bevorzugt in der Pulverkomponente oder in mindestens einer pastenförmigen Komponente des Polymethylmethacrylat-Knochenzementes enthalten ist.

Ebenso ist Gegenstand der Erfindung ein Knochenzement umfassend
(v) als Komponente **1** Daptomycin, ein pharmakologisch verträgliches Salz von Daptomycin, ein Solvat und/oder ein Hydrat enthaltend Daptomycin, und (vi) a) als Komponente **2** das in Wasser lösliche Calciumsalz mit einer Löslichkeit in Wasser bei Raumtemperatur von größer gleich 5 g/l sowie b) als Komponente **3** das in Wasser gering lösliche Calciumsalz mit einer Löslichkeit in Wasser bei Raumtemperatur von kleiner 5 g/l, jeweils unabhängig in Form von Partikeln der Komponenten **1, 2** oder **3** oder jeweils unabhängig als eine partikuläre Formulierung enthalten jeweils unabhängig mindestens eine der Komponenten **1, 2** und/oder **3** und mindestens ein pharmakologisches Hilfsmittel vorliegen und optional jeweils unabhängig eine mittlere Partikelgröße von 1 bis 250 µm, bestimmt mittels Sedimentationsanalyse, aufweisen.

Polymethylmethacrylat-Knochenzemente, die aus zwei lagerstabilen, pastenförmigen Komponenten bestehen, die erst unmittelbar vor der Anwendung zu einem Knochenzementteig vermischt werden, wurden in DE102007050762, DE102008030312 und DE102007052116 beschrieben. Bei diesen Knochenzementen werden zwei Zementpasten separat in geeigneten Kartuschen gelagert. Diese enthalten jeweils neben mindestens einem Monomer und geeigneten Polymeren Bestandteile eines Redoxinitiatorsystems. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Komponenten der Redoxinitiatorsysteme zusammenwirken. Deshalb werden die einzelnen Komponenten des Redoxinitiatorsystems in den separaten Zementpasten so angeordnet, dass diese keine radikalische Polymerisation auslösen können.

Nach einer Ausführungsvariante ist Gegenstand der Erfindung ein Knochenzement, wobei mindestens die Komponente A als
(a4) mindestens eine der Komponenten **1, 2** und **3** umfasst, die jeweils unabhängig in Form von Partikeln der Komponenten **1, 2** oder **3** vorliegen oder jeweils unabhängig als eine partikuläre Formulierung vorliegt, die jeweils unabhängig mindestens eine der Komponenten **1, 2** und/oder **3** enthält, und optional jeweils unabhängig eine mittlere Partikelgröße von 1 bis 250 µm, bestimmt mittels Sedimentationsanalyse, aufweisen,
   wobei die Komponente B als Paste vorliegt und als
(b4) mindestens eine der Komponenten **1, 2** und **3** umfasst, die jeweils unabhängig in Form von Partikeln der Komponenten **1, 2** oder **3** oder jeweils unabhängig als eine partikuläre Formulierung enthalten jeweils unabhängig mindestens eine der Komponenten **1, 2** und/oder **3** vorliegen und optional jeweils unabhängig eine mittlere Partikelgröße von 1 bis 250 µm, bestimmt mittels Sedimentationsanalyse aufweisen.

Besonders bevorzugt sind die Partikel der Komponenten **1, 2** und **3** jeweils als Kombination in einer partikulären Formulierung enthalten, die sowohl in der Komponente A und/oder B enthalten sein kann.

Entsprechend einer Ausführungsvariante umfasst
a) der Knochenzement als (a4) und/oder (b4) Partikel einer Kombination mindestens der Komponenten **1** und **2** oder jeweils unabhängig eine partikuläre Formulierung enthaltend mindestens eine Kombination der Komponenten **1** und **2** und mindestens ein pharmakologisches Hilfsmittel, oder
b) der Knochenzement als (a4) und/oder (b4) Partikel einer Kombination der Komponenten **1, 2** und **3** oder jeweils unabhängig eine partikuläre Formulierung enthalten die Kombination der Komponenten **1, 2** und **3** und mindestens ein pharmakologisches Hilfsmittel.

Vorzugsweise ist das (vi) mindestens eine Calciumsalz, insbesondere das in Wasser lösliche Calciumsalz mit einer Löslichkeit in Wasser bei Raumtemperatur von größer gleich 5 g/l, ausgewählt aus mindestens einem Calcium-Salz einer Zuckersäure, einem Salz einer Carbonsäure mit 1 bis 10 C-Atomen, insbesondere der Milchsäure, einem Salz einer Hydroxycarbonsäure mit 1 bis 10 C-Atomen, einem Salz einer Fruchtsäure, Salzen der Monosaccharide, Salzen der Disaccharide und/oder der jeweiligen Derivate, Calciumchlorid, vorzugsweise CaCl₂ oder einem Hydrat davon.

Nach besonders bevorzugten Ausführungsvarianten umfasst der Knochenzement als a) Komponente **2** das in Wasser lösliche Calciumsalz mit einer Löslichkeit in Wasser bei Raumtemperatur von größer gleich 5 g/l, ein Calciumsalz umfassend Calciumglukonat, Calciumglucuronat, Calciumlactat, Calciumacetat und/oder Calciumsorbat oder eine Mischung enthaltend mindestens zwei der genannten Calciumsalze.

Vorzugsweise umfasst das (vi) mindestens eine Calciumsalz, insbesondere das in Wasser gering lösliche Calciumsalz mit einer Löslichkeit in Wasser bei Raumtemperatur von kleiner 5 g/l ein Calciumsalz umfassend mindestens ein anorganisches Anion umfassend Sulfate, Phosphate oder Salze von Fettsäuren mit 6 bis 31 C-Atomen.

Entsprechend einer weiteren besonders bevorzugten Ausführungsvariante umfasst der Knochenzement als b) Komponente **3** das in Wasser gering lösliche Calciumsalz mit einer Löslichkeit in Wasser bei Raumtemperatur von kleiner 5 g/l, umfassend Calciumsulfat-Dihydrat, Calciumsulfat-Hemihydrat, alpha-Tricalciumphosphat und/oder beta-Tricalciumphosphat.

Der erfindungsgemäße Knochenzement kann zusätzlich zu Daptomycin mindestens ein zweites Antibiotikum enthalten, das ausgewählt ist aus der Gruppe der Aminoglykosid-Antibiotika und/oder der Lincosamid-Antibiotika und/oder der Ansamycin-Antibiotika und/oder der Fluorchinolon-Antibiotika und/oder der β-Lactamantibiotika. Vorzugsweise ist das weitere Antibiotikum ausgewählt aus Gentamicin, Tobramycin und Clindamycin. Die Antibiotika können breitere antibakterielle Wirkungsspektren durch einen verbreiterten antimikrobiellen Schutzumfang des antibiotischen Polymethylmethacrylat-Knochenzementes bewirken. Diese Antibiotika greifen andere Targets der Bakterienzellen an als Daptomycin und vergrößern dadurch die Wahrscheinlichkeit der antimikrobiellen Wirksamkeit des Polymethylmethacrylat-Knochenzements.

Gleichfalls Gegenstand der Erfindung ist ein Verfahren zur Herstellung eines härtbaren Knochenzementes oder eines lokal freisetzenden Wirkstoffträgers sowie der polymerisierbaren Knochenzemente und der Wirkstoffträger erhältlich nach dem Verfahren, indem die Komponenten A und B gemischt werden. Zur Herstellung des lokal freisetzenden Wirkstoffträgers wird der polymerisierbare Knochenzement geformt und polymerisiert.

Der erfindungsgemäße Knochenzement dient insbesondere als Revisionszement, zur Anwendung in einem Verfahren zur Behandlung und/oder Prävention von Infektionen die durch Bakterien ausgelöst werden, insbesondere zur Behandlung und/oder Prävention einer Infektion durch bakterielle Multi-Resistente-Erreger (MRE-Keime).

Die Verwendung des Knochenzementes oder eines Wirkstoffträgers zur Behandlung und/oder Prävention von durch Bakterien ausgelösten Infektionen umfasst besonders bevorzugt die Behandlung und/oder Prävention einer Infektion durch bakterielle Multi-Resistente-Erreger (MRE-Keime), wie VRSA, MRSA, VRE usw. Als MRE-Keime gelten: Methicillin-resistente Staphylococcus aureus (MRSA)-Stämme, Vancomycin-intermediärsensible Staphylococcus aureus (VISA)-Stämme, Vancomycin-resistente Staphylococcus aureus (VRSA)-Stämme, Extended Spectrum β-Lactamase (ESBL) produzierende Erreger Mehrfach-resistente Gram-positive Bakterien (MRGP/MDRGP) können Vancomycin/Glykopeptid-resistente Enterokokken (VRE, GRE), Penicillin-resistente Pneumokokken, etc. umfassen. Mehrfach-resistente Gram-negative Bakterien (MRGN/MDRGN) können u.a. Pseudomonas aeruginosa: Bakterium Acinetobacter baumannii als Verursacher von Wundinfektionen und Sepsis umfassen.

Gegenstand der Erfindung ist ferner ein Verfahren zur Herstellung eines härtbaren Knochenzementes oder eines lokal freisetzenden Wirkstoffträgers, indem zwei Komponenten A und B gemischt werden, wobei
Komponente A als Paste vorliegt und umfasst
(a1) mindestens ein radikalisch polymerisierbares Monomer,
(a2) mindestens ein organisches Polymer umfassend mindestens ein Polymethylmethacrylat und/oder ein Polymethylmethacrylat-Copolymer, und
(a3) mindestens einen Polymerisationsinitiator; und
Komponente B als Paste vorliegt und umfasst
(b1) mindestens ein radikalisch polymerisierbares Monomer,
(b2) mindestens ein organisches Polymer umfassend mindestens ein Polymethylmethacrylat und/oder ein Polymethylmethacrylat-Copolymer, und
(b3) mindestens einen Polymerisationsbeschleuniger, dadurch gekennzeichnet, dass als Komponente **1** Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l zugemischt werden, wobei
die Komponente A
als (a4) mindestens eine der Komponenten **1, 2** und **3** umfasst, wobei Komponente **1** ausgewählt ist aus Daptomycin, einem pharmakologisch verträglichen Salz von Daptomycin, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l, und
die Komponente B
als (b4) mindestens eine der Komponenten **1, 2** und **3** umfasst, wobei Komponente **1** ausgewählt ist aus Daptomycin, einem pharmakologisch verträglichen Salz von Daptomycin, einer polymorphen Form des Daptomycins, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l.

Gegenstand der vorliegenden Erfindung ist ferner ein Verfahren zur Herstellung eines härtbaren Knochenzementes oder eines lokal freisetzenden Wirkstoffträgers nach einem der Ansprüche 1 bis 8, indem zwei Komponenten A und B gemischt werden, wobei Komponente A als Pulver vorliegt und umfasst
(a1) mindestens ein pulverförmiges Polymer umfassend mindestens ein Polymethylmethacrylat und/oder ein pulverförmiges Gemisch umfassend Polymethylmethacrylat-Co-Polymere,
(a2) mindestens einen pulverförmigen Röntgenopaker, und
(a3) mindestens einen Polymerisationsinitiator; und
Komponente B als Flüssigkeit oder Paste vorliegt und umfasst
(b1) mindestens ein radikalisch polymerisierbares Monomer,
(b2) optional mindestens ein organisches Polymer umfassend mindestens ein Polymethylmethacrylat und/oder ein Polymethylmethacrylat-Copolymer, und
(b3) mindestens einen Polymerisationsbeschleuniger; dadurch gekennzeichnet, dass als Komponente **1** Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l zugemischt werden,
wobei mindestens die Komponente A
als (a4) mindestens eine der Komponenten **1, 2** und **3** umfasst, die ausgewählt ist aus Komponente **1** Daptomycin, einem pharmakologisch verträglichen Salz von Daptomycin, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente **3** einem in Wasser gering löslichem Calciumsalz mit einer Löslichkeit in Wasser bei Raumtemperatur von kleiner 5 g/l, und/oder
wobei die Komponente B als Paste vorliegt und
als (b4) mindestens eine der Komponenten **1, 2** und **3** umfasst, die ausgewählt ist aus Komponente **1** Daptomycin, einem pharmakologisch verträglichen Salz von Daptomycin, einer polymorphen Form des Daptomycins, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente **3** einem in Wasser gering löslichem Calciumsalz mit einer Löslichkeit in Wasser bei Raumtemperatur von kleiner 5 g/l.

Dabei können die Komponenten **1, 2** oder **3** jeweils unabhängig als eine partikuläre Formulierung enthalten sein, oder sie können in einer Kombination von jeweils 2 Komponenten oder allen drei Komponenten in einer partikulären Formulierung im Kit enthalten sein. Optional kann das Kit mindestens ein pharmakologisches Hilfsmittel enthalten.

Ein aus dem erfindungsgemäßen Knochenzement polymerisierter, gehärteter Knochenzement kann zur Anwendung in einem Verfahren zur Behandlung und/oder Prävention von Infektionen die durch Bakterien ausgelöst werden, insbesondere zur Behandlung und/oder Prävention einer Infektion durch bakterielle Multi-Resistente-Erreger dienen, wobei die Komponente **1** Daptomycin, ein pharmakologisch verträgliches Salz von Daptomycin, eine polymorphe Form des Daptomycins, ein Solvat und/oder ein Hydrat enthaltend Daptomycin, und mindestens ein Calcium-Salz, vorzugsweise mindestens zwei in Wasser unterschiedlich lösliche Calcium-Salze als Komponenten **2** und **3** enthält. Besonders bevorzugt ist ein Calciumsalz schnellfreisetzend und wird das mindestens eine weitere Calciumsalz retardiert freigesetzt, wobei die Freisetzung in Gegenwart von Feuchte, Wasser, wässrigem Milieu, wie Körperflüssigkeiten, oder einer wässrigen Lösung erfolgt. Vorzugsweise sind die Komponenten 2 und 3 ausgewählt aus einer Komponente **2,** einem in Wasser löslichen Calciumsalz mit einer Löslichkeit in Wasser bei Raumtemperatur von größer gleich 5 g/l, und der Komponente **3,** einem in Wasser gering löslichen Calciumsalz mit einer Löslichkeit in Wasser bei Raumtemperatur von kleiner 5 g/l.

Gegenstand der Erfindung ist auch ein Formkörper erhältlich durch Formen und Polymerisation des polymerisierbaren Knochenzements.

Ebenso Gegenstand der Erfindung ist ein Formkörper zur Anwendung in einem Verfahren zur Behandlung und/oder Prävention von Infektionen, die durch Bakterien ausgelöst werden, insbesondere durch Multi-Resistente-Erreger. Beispiele sind ein chirurgisches Implantat oder ein Teil eines Implantates, ein antibiotisches Implantat, ein Revisionsimplantat, eine Schraube, ein Nagel, eine chirurgische Platte. Erfindungsgemäß dienen die Implantate zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

Das (i) Monomer ist vorzugsweise ausgewählt aus mindestens einem Alkyl-2-acrylsäurealkylester, Aryl-2-acrylsäurealkylester, Arylalkyl-2-acrylsäurealkylester, jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Monomere, und/oder das (ii) organische Polymer ist vorzugsweise ausgewählt aus mindestens einem Poly(alkyl-2-acrylsäure-alkylester), Poly(aryl-2-acrylsäurealkylester), Poly(arylalkyl-2-acrylsäurealkylester), jeweils unabhängig mit 1 bis 20 C-Atomen in der Alkyl-Gruppe, jeweils unabhängig mit 6 bis 14 C-Atomen in der Aryl-Gruppe, jeweils unabhängig mit 6 bis 14 C-Atomen in der Arylalkyl-Gruppe und jeweils unabhängig mit 1 bis 10 C-Atomen in der Alkylester-Gruppe oder einer Mischung umfassend mindestens zwei der genannten Polymere.

Ein erfindungsgemäßer Knochenzement kann neben dem organischen Polymer, insbesondere Polymethmethacrylat (PPMA), und dem radikalisch polymerisierbaren Monomer, insbesondere Methacrylsäuremethylester, ein partikuläres, anorganisches Additiv umfassen, vorzugsweise in einer Konzentration von 0,01 bis 0,5 Gew.-%, insbesondere von 0,01 bis 0,25 Gew.-%, bevorzugt von 0,02 bis 0,14 Gew.-% in Bezug auf die Gesamtzusammensetzung. Erfindungsgemäss umfasst der aus der Pulverkomponente und der flüssigen Monomerkomponente gemischte Knochenzementteig das partikuläre, anorganische Additiv in einer Konzentration von 0,02 bis 0,14 Gew.-%. Zusätzlich zu den
vorgenannten Komponenten umfasst ein erfindungsgemäßer Knochenzement einen Röntgenopaker, einen Polymerisationsinitiator und/oder einen Polymerisationsbeschleuniger sowie optional zusätzlich Füllstoffe, die nicht dem Additiv entsprechen und lediglich verdickende Wirkung zeigen.

Das partikuläre anorganische Additiv kann ausgewählt sein aus der Gruppe aus pyrogenem Siliziumdioxid, pyrogenem Metall-Siliziummischoxid, Bentonit, Montmorillonit und einer Mischung enthaltend mindestens zwei der genannten Additive. Daneben ist es auch möglich, hydrophobiertes pyrogenes Siliziumdioxid einzusetzen.

Der erfindungsgemäße Knochenzement, die Pasten, Flüssigkeit und/oder Pulverkomponenten können mindestens einen Polymerisationsinitiator (der vorzugsweise in dem radikalisch polymerisierbaren Monomer löslich ist), wenigstens einen Polymerisationsbeschleuniger (der vorzugsweise in dem radikalisch polymerisierbaren Monomer löslich ist), gegebenenfalls wenigstens einen Co-Polymerisationsbeschleuniger oder wenigstens einen Polymerisationsinitiator, wenigstens einen Polymerisationsbeschleuniger und gegebenenfalls wenigstens einen Co-Polymerisationsbeschleuniger beinhalten.

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden erfindungsgemäß Verbindungen verstanden, die wenigstens eine Peroxogruppe (-O-O-) enthalten. Das Peroxid weist vorzugsweise keine freien aciden Gruppen auf. Bei dem Peroxid kann es sich um ein anorganisches Peroxid oder um ein organisches Peroxid handeln, wie zum Beispiel ein toxikologisch unbedenkliches Hydroperoxid. Gemäss einer besonders bevorzugten Ausführungsform ist das Peroxid aus der Gruppe ausgewählt, die aus Cumol-hydroperoxid, 1,1,3,3-Tetramethylbutyl-hydroperoxid, t-Butyl-hydroperoxid, t-Amylhydroperoxid, Di-Isopropylbenzen-mono-hydroperoxid und einer Mischung aus mindestens zwei davon besteht. Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten und 1,3,5-trisubstituierten Barbituraten. Gemäss einer besonderen Ausgestaltung der erfindungsgemässen Paste ist das Barbitursäurederivat ausgewählt aus der Gruppe bestehend aus 1,5-disubstituierten Barbituraten und 1,3,5-trisubstituierten Barbituraten. Es können vorzugsweise 1,5-disubstituierte Thiobarbiturate oder 1,3,5-trisubstituierte Thiobarbiturate eingesetzt werden. Gemäß einer bevorzugten Ausführungsform weisen die Substituenten jeweils eine Länge von 1 bis 10 Kohlenstoffatomen auf. Gemäß einer besonders bevorzugten Ausführungsform ist das Barbitursäurederivat aus der Gruppe aus 1-Cyclohexyl-5-ethyl-barbitursäure, 1-Phenyl-5-ethyl-barbitursäure und 1,3,5-Trimethyl-barbitursäure ausgewählt.

Polymerisationsinitiator und Polymerisationsbeschleuniger können jeweils unabhängig von einander in der Komponente A und/oder B zu 0,01 bis 5 Gew.-% enthalten sein.

Als Polymerisationsbeschleuniger sind Schwermetallverbindungen, die aus der Gruppe bestehend aus Schwermetallsalzen und Schwermetallkomplexen ausgewählt sind, bevorzugt. Erfindungsgemäß bevorzugte Schwermetallverbindungen sind ausgewählt aus der Gruppe bestehend aus Kupfer(II)-hydroxid, Kupfer(II)methacrylat, Kupfer(II)acetyIacetonat, Kupfer(II)-2-ethyl-hexanoat, Cobalt(II)-hydroxid, Cobalt(II)-2-ethyl-hexanoat, basischem Kupfer(II)-carbonat, Eisen(II)-2-ethyl-hexanoat, Eisen(III)-2-ethyl-hexanoat und einer Mischung aus mindestens zwei davon.

Gemäß einer anderen Ausgestaltungsform kann der Knochenzement oder mindestens eine Paste, Flüssigkeit oder Pulverkomponente einen Polymerisationsbeschleuniger umfassen, der ausgewählt aus der Gruppe bestehend aus N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid, Pyromelithsäuredümid und einer Mischung aus mindestens zwei davon.

Eine weitere vorteilhafte Ausgestaltung der Erfindung umfasst eine Verwendung von Kombinationen aus Schwermetallsalzen und mindestens einem Vertreter aus der Gruppe umfassend N,N-Dimethyl-p-toluidin, N,N-Bis-hydroxyethyl-p-toluidin, N,N-Dimethyl-anilin, Trioctylmethylammoniumchlorid, Tetrabutylammoniumchlorid, Lithiumchlorid, Saccharin, 1,8-Diazabicyclo[5.4.0]undec-7-en und 1,5-Diazabicyclo(4.3.0)non-5-en, Phthalimid, Maleimid, Succinimid und Pyromelithsäurediimid als Polymerisationsbeschleuniger. Dabei werden Zweifachkombinationen und auch Dreifachkombinationen von unterschiedlichen Polymerisationsbeschleunigern im Rahmen der Erfindung offenbart.

Eine vorteilhafte Ausgestaltung der Erfindung besteht darin, dass gegebenenfalls wenigstens ein Co-Polymerisationsbeschleuniger in der erfindungsgemäßen Zusammensetzung oder in einer der Pasten A, B oder der Flüssigkeit B oder Pulverkomponente A enthalten ist, wobei als Co-Polymerisationsbeschleuniger tertiäre Amine und Amidine bevorzugt sind, und wobei ganz besonders N,N-Dimethyl-p-toluidin, N,N-Bis-hydroxyethyl-p-toluidin, N,N-Dimethylanilin, 1,8-Diazabicyclo[5.4.0-]undec-7-en und 1,5-Diazabicyclo(4.3.0)-non-5-en als Co-Akzeleratoren/Beschleuniger bevorzugt sind.

Der erfindungsgemäße Knochenzement, insbesondere in Form einer Paste kann den Polymerisationsinitiator, den Polymerisationsbeschleuniger, den Co-Polymerisationsbeschleuniger oder den Polymerisationsinitiator, den Polymerisationsbeschleuniger und den Co-Polymerisationsbeschleuniger in einer (Gesamt)Menge von bis zu 10 Gew.-%, vorzugsweise 1 bis 5 Gew.-%, bezogen auf das Gesamtgewicht des Knochenzementes oder jeweils unabhängig bezogen auf das Gesamtgewicht einer der Pasten A, B, Flüssigkeit B oder Pulverkomponente A, beinhalten.

Der erfindungsgemäße Knochenzement, insbesondere in Form einer Paste oder die Pasten A, B oder Flüssigkeit B als auch die Pulverkomponente A können neben den vorstehend genannten Komponenten weitere Bestandteile beinhalten.

Das Mischungsverhältnis von Komponente A zu B beträgt in der Regel 1 : 10 bis 10: 1 Vol.-%, vorzugsweise 1 : 2 bis 2 : 1 Vol.-%.

Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen bestehen. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht. Die Konzentrationen an Röntgenopaker, insbesondere die Konzentration an Zirkoniumdioxid, in dem Knochenzement oder einer der Pasten A, B, Flüssigkeit B oder Pulverkomponente A können jeweils voneinander unabhängig beispielsweise in einem Bereich von 3 bis 30 Gew.-% in Bezug auf die jeweilige Gesamtzusammensetzung liegen. Röntgenopaker gelten vorliegend nicht als Füllstoffe.

Der Polymerisationsstabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigen. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Der erfindungsgemäße antibiotische Polymethylmethacrylat-Knochenzement wird als Revisionszement für einzeitige und zweizeitige septische Revisionen, zur Herstellung von Spacern und zur Herstellung von lokal freisetzenden Wirkstoffträgern verwendet. Die lokal freisetzenden Wirkstoffträger können jede geeignete dreidimensionale Form aufweisen, wie kugelförmig, bohnenförmig oder auch stabförmig. Besonders bevorzugt sind Formen, die extrudierbar sind oder in einem Granulations- oder Kompaktierverfahren erhalten werden können.

Die Erfindung wird durch die nachstehenden Beispiele erläutert, ohne jedoch die Erfindung auf diese Beispiele zu beschränken.

### Beispiele

Es wurde zuerst ein Basiszementpulver mit folgender Zusammensetzung durch Vermischung der Komponenten in einer 1,5 l Plastikflasche mit Hilfe eines Turbula-Mischers hergestellt:
88,9 Gew.-% Polymethylmethacrylat-co-methylacrylat (Mw > 400.000 g/ml), 10,0 Gew.-% Zirkoniumdioxid, 1,1 Gew.-% Dibenzoylperoxid.

Die Zementpulver der Beispiele 1-4 wurden durch Vermischung des Basiszementpulvers mit Daptomycin (Aktivitätskoeffizient AK= 943), Gentamicinsulfat (Fukang Fujian, Aktivitätskoeffizient AK= 580), Calciumglukonat (Sigma-Aldrich) und Calciumsulfat-Dihydrat (Sigma-Aldrich) unter Verwendung eines Turbula-Mischers hergestellt.

### Zusammensetzung der Zementpulver der Beispiele 1 bis 4

| Beispiel | Zusammensetzung der Zementpulver [g] | | | | | Gesamtmasse Zementpulver [g] |
|---|---|---|---|---|---|---|
| | Basiszementpulver | Daptomycin | Gentamicinsulfat | Calciumglukonat | Caliumsulfatdihydrat | |
| 1 | 40,0 | 0,50 | 0,86 | 0,50 | 0,50 | 42,36 |
| 2 | 40,0 | 0,75 | 0,86 | 0,50 | 0,50 | 42,61 |
| 3 | 40,0 | 1,00 | 0,86 | 0,50 | 0,50 | 42,86 |
| 4 | 40,0 | 1,50 | 0,86 | 0,50 | 0,50 | 43,36 |

| | | | | | | |
|---|---|---|---|---|---|---|
| Gentamicinsulfat mit einem Aktivitätskoeffizienten von AK= 580 Daptomycin mit einem Aktivitätskoeffizienten von AK= 943 | | | | | | |

Für die nachfolgende Herstellung von Probekörpern wurde jeweils das Zementpulver der Beispiele 1 bis 4 mit jeweils 20 ml Monomerflüssigkeit vermischt. Die Monomerflüssigkeit war jeweils aus 18,50 Methylmethacrylat, 0,38 g N,N-Dimethyl-p-toluidin, 0,002 g Hydrochinon und Spuren von Chlorophyllin (E241) zusammengesetzt. Nach der Vermischung der Zementpulver der Beispiele 1 bis 4 mit jeweils 20 ml Monomerflüssigkeit entstand nach ca. 60 Sekunden ein plastisch verformbarer, grünlicher Zementteig, der zur Herstellung von Prüfkörpern verwendet wurde. Der Zementteig härtete nach ca. 4 Minuten aus.

Es wurden für die Bestimmung der Biegefestigkeit und des Biegemoduls gemäß ISO 5833-E/F:2002 streifenförmige Prüfkörper mit den Abmessungen 3,3 mm x 10,0 mm x 75,0 mm hergestellt. Für die Bestimmung der Druckfestigkeit wurden zylinderförmige Probekörper mit einem Durchmesser von 6,0 mm und mit einer Höhe von 10,0 mm gefertigt. Die Prüfung der Biegefestigkeit, des Biegemoduls und der Druckfestigkeit gemäß der ISO 5833 erfolgte mit einer Zwick-Universalprüfmaschine Z010.

| Beispiel | Biegefestigkeit [MPa] | Biegemodul [MPa] | Druckfestigkeit [MPa] |
|---|---|---|---|
| 1 | 69,9 ± 1,6 | 3120 ± 95 | 91,8 ± 1,3 |
| 2 | 71,9 ± 1,6 | 3251 ± 60 | 93,4 ± 2,6 |
| 3 | 72,3 ± 1,4 | 3342 ± 113 | 90,3 ± 1,9 |
| 4 | 66,0 ± 2,3 | 3148 ± 168 | 93,3 ± 2,8 |

Die Norm ISO 5833 fordert für eine Biegefestigkeit größer 50 MPa, ein Biegemodul größer 1800 MPa und eine Druckfestigkeit von größer 70 MPa. Die Zemente der Beispiele 1 bis 4 erfüllen die Anforderungen der ISO 5833 hinsichtlich der Biegefestigkeit, des Biegemoduls und der Druckfestigkeit.

Für die Prüfung der in vitro-Freisetzung von Daptomycin, Gentamicin und Calcium wurden mit den Zementpulvern der Beispiele 1 bis 4 zylinderförmige Prüfkörper (Durchmesser 25 mm, Höhe 10 mm) hergestellt. Dazu wurden jeweils ein Probekörper in 20 ml einer wässrigen 0,1 M Tris-HCI-Puffer pH 7,4 bei 37 °C über einen Zeitraum von 5 Tagen gelagert. Von jedem Zement wurden drei Prüfkörper parallel eluiert. Jeden Tag wurde das Elutionsmedium vollständig entfernt und durch neues Elutionsmedium ersetzt. Der Daptomycin- und der Gentamicin-Gehalt der Eluate wurde durch HPLC-MS/MS bestimmt (AZB Biopharm GmbH, Berlin, AZB Report: AZB15-025). Die Mittelwerte der Messergebnisse sind in den nachfolgenden Tabellen dargestellt.

| Zeit [d] | Gentamicin [µg/Probekörper] | | | |
|---|---|---|---|---|
| | Beispiel 1 | Beispiel 2 | Beispiel 3 | Beispiel 4 |
| 1 | 446 ± 134 | 536 ± 72 | 655 ± 103 | 734 ± 48 |
| 2 | 169 ± 17 | 184 ± 25 | 192 ± 38 | 327 ± 58 |
| 3 | 105 ± 23 | 130 ± 27 | 138 ± 13 | 346 ± 138 |
| 4 | 76 ± 11 | 80 ± 14 | 94 ± 4 | 177 ± 68 |
| 5 | 56 ± 10 | 80 ± 14 | 89 ± 20 | 107 ± 8 |

| Zeit [d] | Daptomycin [µg/Probekörper] | | | |
|---|---|---|---|---|
| | Beispiel 1 | Beispiel 2 | Beispiel 3 | Beispiel 4 |
| 1 | 264 ± 28 | 447 ± 22 | 611 ± 27 | 1040 ± 31 |
| 2 | 18 ± 3 | 27 ± 3 | 42 ± 6 | 95 ± 8 |
| 3 | 10 ± 1 | 16 ± 3 | 24 ± 5 | 43 ± 8 |
| 4 | 9 ± 2 | 14 ± 4 | 19 ± 4 | 43 ± 5 |
| 5 | 6 ± 1 | 9 ± 1 | 16 ± 3 | 28 ± 2 |

Die Bestimmung der Calcium-Konzentration in den Eluaten wurde mit ICP-MS vorgenommen.

| Zeit [d] | Calcium [µg/Probekörper] | | | |
|---|---|---|---|---|
| | Beispiel 1 | Beispiel 2 | Beispiel 3 | Beispiel 4 |
| 1 | 86 ± 6 | 97 ± 2 | 102 ± 2 | 122 ± 1 |
| 2 | 12 ± 1 | 13 ± 0 | 15 ± 1 | 21 ± 1 |
| 3 | 7 ± 1 | 8 ± 0 | 9 ± 1 | 13 ± 0 |
| 4 | 5 ± 0 | 7 ± 1 | 7 ± 1 | 10 ± 1 |
| 5 | 4 ± 0 | 5 ± 0 | 6 ± 0 | 8 ± 0 |

Es wurden weiterhin zur Prüfung der antimikrobiellen Wirksamkeit zylinderförmige Prüfkörper (Durchmesser 6 mm, Höhe 15 mm) mit den Zementpulvern der Beispiele 1 bis 4 unter Verwendung der Monomerflüssigkeit hergestellt. Mit diesen Prüfkörpern wurde ein CERTIKA-Proliferationstest unter Verwendung des Testkeims *Staphylococcus aureus* CCUG 45315 (VRSA) durchgeführt. Die Prüfkörper der Zemente der Beispiele 1 bis 4 zeigten eine vollständige Hemmung des Testkeims im CERTIKA-Proliferationstest (Quality Labs GmbH Nürnberg, Report zu Work Order 1935, Measurement 20140925-R06-01-10).

## Patentansprüche

1. Antibiotischer, polymerisierbarer Knochenzement umfassend
(i) mindestens ein radikalisch polymerisierbares Monomer
(ii) mindestens ein organisches Polymer umfassend mindestens ein Polymethylmethacrylat und/oder ein Polymethylmethacrylat-Copolymer, und
(iii) mindestens einen Polymerisationsinitiator,
(iv) mindestens einen Röntgenopaker,
**dadurch gekennzeichnet, dass**
der Knochenzement umfasst
(v) als Komponente **1** Daptomycin, ein pharmakologisch verträgliches Salz von Daptomycin, eine polymorphe Form des Daptomycins, ein Solvat und/oder ein Hydrat enthaltend Daptomycin, **dadurch gekennzeichnet, dass**
der Knochenzement ferner umfasst
(vi) eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l.

2. Knochenzement nach Anspruch 1, **dadurch gekennzeichnet, dass**
(v) die Komponente **1** Daptomycin, ein pharmakologisch verträgliches Salz von Daptomycin, eine polymorphe Form des Daptomycins, ein Solvat und/oder ein Hydrat enthaltend Daptomycin, und (vi) a) die Komponente **2** das in Wasser lösliche Calciumsalz mit einer Löslichkeit in Wasser bei Raumtemperatur von größer gleich 5 g/l sowie b) die Komponente **3** das in Wasser gering lösliche Calciumsalz mit einer Löslichkeit in Wasser bei Raumtemperatur von kleiner 5 g/l, jeweils unabhängig in Form von Partikeln der Komponenten **1, 2** oder **3** vorliegen oder jeweils unabhängig als eine partikuläre Formulierung enthaltend jeweils unabhängig mindestens eine der Komponenten **1, 2** und/oder **3** und mindestens ein pharmakologisches Hilfsmittel vorliegen.

3. Knochenzement nach einem der Ansprüche 1 oder 2, **dadurch gekennzeichnet, dass** mindestens die Komponente A
als (a4) mindestens eine der Komponenten **1, 2** und **3** umfasst, die jeweils unabhängig in Form von Partikeln der Komponenten **1, 2** oder **3** oder jeweils unabhängig als eine partikuläre Formulierung enthaltend jeweils unabhängig mindestens eine der Komponenten **1, 2** und/oder **3** vorliegt,
wobei die Komponente B als Paste vorliegt und
als (b4) mindestens eine der Komponenten **1, 2** und **3** umfasst, die jeweils unabhängig in Form von Partikeln der Komponenten **1, 2** oder **3** oder jeweils unabhängig als eine partikuläre Formulierung enthaltend jeweils unabhängig mindestens eine der Komponenten **1, 2** und/oder **3** vorliegt.

4. Knochenzement nach einem der Ansprüche 1 bis 3, **dadurch gekennzeichnet, dass**
a) der Knochenzement als (a4) und/oder (b4) als Partikel einer Kombination mindestens der Komponenten **1** und **2** oder jeweils unabhängig als eine partikuläre Formulierung enthaltend mindestens eine Kombination der Komponenten **1** und **2** und mindestens ein pharmakologisches Hilfsmittel vorliegt, oder
b) der Knochenzement als (a4) und/oder (b4) als Partikel einer Kombination der Komponenten **1, 2** und **3** oder jeweils unabhängig als eine partikuläre Formulierung enthaltend die Kombination der Komponenten **1, 2** und **3** und mindestens ein pharmakologisches Hilfsmittel vorliegt.

5. Knochenzement nach einem der Ansprüche 1 bis 4, **dadurch gekennzeichnet, dass** mindestens eine der Komponenten **1, 2** und/oder **3** eine mittlere Partikelgröße von 1 bis 250 µm aufweist.

6. Knochenzement nach einem der Ansprüche 1 bis 5, **dadurch gekennzeichnet, dass**
a) die Komponente **2,** das in Wasser lösliche Calciumsalz mit einer Löslichkeit in Wasser bei Raumtemperatur von größer gleich 5 g/l umfasst Calciumglukonat, Calciumglucuronat, Calciumlactat, Calciumacetat und/oder Calciumsorbat oder eine Mischung enthaltend mindestens zwei der genannten Calciumsalze.

7. Knochenzement nach einem der Ansprüche 1 bis 6, **dadurch gekennzeichnet, dass**
b) die Komponente **3** das in Wasser gering lösliches Calciumsalz mit einer Löslichkeit in Wasser bei Raumtemperatur von kleiner 5 g/l umfasst Calciumsulfat-Dihydrat, Calciumsulfat-Hemihydrat, alpha-Tricalciumphosphat und/oder beta-Tricalciumphosphat.

8. Knochenzement nach einem der Ansprüche 1 bis 7, **dadurch gekennzeichnet, dass** der Knochenzement mindestens ein zweites Antibiotikum enthält, ausgewählt aus der Gruppe der Aminoglykosid-Antibiotika und/oder der Lincosamid-Antibiotika und/oder der Ansamycin-Antibiotika und/oder der Fluorchinolon-Antibiotika und/oder der β-Lactamantibiotika.

9. Knochenzement-Kit zur Herstellung eines Knochenzementes nach einem der Ansprüche 1 bis 8, enthaltend
zwei Komponenten A und B, wobei
Komponente A als Paste vorliegt und umfasst
(a1) mindestens ein radikalisch polymerisierbares Monomer,
(a2) mindestens ein organisches Polymer umfassend mindestens ein Polymethylmethacrylat und/oder ein Polymethylmethacrylat-Copolymer, und
(a3) mindestens einen Polymerisationsinitiator; und
Komponente B als Paste vorliegt und umfasst
(b1) mindestens ein radikalisch polymerisierbares Monomer,
(b2) mindestens ein organisches Polymer umfassend mindestens ein Polymethylmethacrylat und/oder ein Polymethylmethacrylat-Copolymer, und
(b3) mindestens einen Polymerisationsbeschleuniger, **dadurch gekennzeichnet, dass** das Kit umfasst:
als Komponente **1** Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l, wobei
die Komponente A
als (a4) mindestens eine der Komponenten **1, 2** und **3** umfasst, wobei Komponente **1** ausgewählt ist aus Daptomycin, einem pharmakologisch verträglichen Salz von Daptomycin, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l, und
die Komponente B
als (b4) mindestens eine der Komponenten **1, 2** und **3** umfasst, wobei Komponente **1** ausgewählt ist aus Daptomycin, einem pharmakologisch verträglichen Salz von Daptomycin, einer polymorphen Form des Daptomycins, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l.

10. Knochenzement-Kit zur Herstellung eines Knochenzementes nach einem der Ansprüche 1 bis 8, enthaltend
zwei Komponenten A und B, wobei
Komponente A als Pulver vorliegt und umfasst
(a1) mindestens ein pulverförmiges Polymer umfassend mindestens ein Polymethylmethacrylat und/oder ein pulverförmiges Gemisch umfassend Polymethylmethacrylat-Co-Polymere,
(a2) mindestens einen pulverförmigen Röntgenopaker, und
(a3) mindestens einen Polymerisationsinitiator; und
Komponente B als Flüssigkeit oder Paste vorliegt und umfasst
(b1) mindestens ein radikalisch polymerisierbares Monomer,
(b2) optional mindestens ein organisches Polymer umfassend mindestens ein Polymethylmethacrylat und/oder ein Polymethylmethacrylat-Copolymer, und
(b3) mindestens einen Polymerisationsbeschleuniger; **dadurch gekennzeichnet, dass** das Kit umfasst:
als Komponente **1** Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l, wobei
mindestens die Komponente A
als (a4) mindestens eine der Komponenten **1, 2** und **3** umfasst, die ausgewählt ist aus Komponente **1** Daptomycin, einem pharmakologisch verträglichen Salz von Daptomycin, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente **3** einem in Wasser gering löslichem Calciumsalz mit einer Löslichkeit in Wasser bei Raumtemperatur von kleiner 5 g/l, und/oder
wobei die Komponente B als Paste vorliegt und
als (b4) mindestens eine der Komponenten **1, 2** und **3** umfasst, die ausgewählt ist aus Komponente **1** Daptomycin, einem pharmakologisch verträglichen Salz von Daptomycin, einer polymorphen Form des Daptomycins, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente 3 einem in Wasser gering löslichem Calciumsalz mit einer Löslichkeit in Wasser bei Raumtemperatur von kleiner 5 g/l.

11. Verfahren zur Herstellung eines härtbaren Knochenzementes oder eines lokal freisetzenden Wirkstoffträgers nach einem der Ansprüche 1 bis 8, indem zwei Komponenten A und B gemischt werden, wobei
Komponente A als Paste vorliegt und umfasst
(a1) mindestens ein radikalisch polymerisierbares Monomer,
(a2) mindestens ein organisches Polymer umfassend mindestens ein Polymethylmethacrylat und/oder ein Polymethylmethacrylat-Copolymer, und
(a3) mindestens einen Polymerisationsinitiator; und
Komponente B als Paste vorliegt und umfasst
(b1) mindestens ein radikalisch polymerisierbares Monomer,
(b2) mindestens ein organisches Polymer umfassend mindestens ein Polymethylmethacrylat und/oder ein Polymethylmethacrylat-Copolymer, und
(b3) mindestens einen Polymerisationsbeschleuniger, **dadurch gekennzeichnet, dass** als Komponente **1** Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l zugemischt werden, wobei
die Komponente A
als (a4) mindestens eine der Komponenten **1, 2** und **3** umfasst, wobei Komponente **1** ausgewählt ist aus Daptomycin, einem pharmakologisch verträglichen Salz von Daptomycin, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l, und
die Komponente B
als (b4) mindestens eine der Komponenten **1, 2** und **3** umfasst, wobei Komponente **1** ausgewählt ist aus Daptomycin, einem pharmakologisch verträglichen Salz von Daptomycin, einer polymorphen Form des Daptomycins, einem Solvat und/oder einem Hydrat enthaltend Daptomycin, Komponente **2** ausgewählt ist aus einem in Wasser löslichem Calciumsalz mit einer Löslichkeit in Wasser bei Raumtemperatur von größer gleich 5 g/l und Komponente **3** ausgewählt ist aus einem in Wasser gering löslichem Calciumsalz mit einer Löslichkeit in Wasser bei Raumtemperatur von kleiner 5 g/l.

12. Verfahren zur Herstellung eines härtbaren Knochenzementes oder eines lokal freisetzenden Wirkstoffträgers nach einem der Ansprüche 1 bis 8, indem zwei Komponenten A und B gemischt werden, wobei
Komponente A als Pulver vorliegt und umfasst
(a1) mindestens ein pulverförmiges Polymer umfassend mindestens ein Polymethylmethacrylat und/oder ein pulverförmiges Gemisch umfassend Polymethylmethacrylat-Co-Polymere,
(a2) mindestens einen pulverförmigen Röntgenopaker, und
(a3) mindestens einen Polymerisationsinitiator; und
Komponente B als Flüssigkeit oder Paste vorliegt und umfasst
(b1) mindestens ein radikalisch polymerisierbares Monomer,
(b2) optional mindestens ein organisches Polymer umfassend mindestens ein Polymethylmethacrylat und/oder ein Polymethylmethacrylat-Copolymer, und
(b3) mindestens einen Polymerisationsbeschleuniger; **dadurch gekennzeichnet, dass** als Komponente 1 Daptomycin, ein pharmakologisch verträgliches Salz von Daptomycin, eine polymorphe Form des Daptomycins, ein Solvat und/oder ein Hydrat enthaltend Daptomycin, und
eine Kombination enthaltend
a) als Komponente 2 ein in Wasser lösliches Calciumsalz mit einer Löslichkeit in Wasser bei Raumtemperatur von größer gleich 5 g/l, und
b) als Komponente 3 ein in Wasser gering lösliches Calciumsalz mit einer Löslichkeit in Wasser bei Raumtemperatur von kleiner 5 g/l zugemischt werden,
wobei mindestens die Komponente A
als (a4) mindestens eine der Komponenten **1, 2** und **3** umfasst, die ausgewählt ist aus Komponente **1** Daptomycin, einem pharmakologisch verträglichen Salz von Daptomycin, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente **3** einem in Wasser gering löslichem Calciumsalz mit einer Löslichkeit in Wasser bei Raumtemperatur von kleiner 5 g/l, und/oder
wobei die Komponente B als Paste vorliegt und
als (b4) mindestens eine der Komponenten **1, 2** und **3** umfasst, die ausgewählt ist aus Komponente **1** Daptomycin, einem pharmakologisch verträglichen Salz von Daptomycin, einer polymorphen Form des Daptomycins, einem Solvat und/oder einem Hydrat enthaltend Daptomycin, der Komponente **2** einem in Wasser löslichem Calciumsalz mit einer Löslichkeit in Wasser bei Raumtemperatur von größer gleich 5 g/l und der Komponente **3** einem in Wasser gering löslichem Calciumsalz mit einer Löslichkeit in Wasser bei Raumtemperatur von kleiner 5 g/l.

13. Lokal freisetzender Wirkstoffträger erhältlich nach einem Verfahren nach einem der Ansprüche 11 oder 12.

14. Formkörper erhältlich durch Formen und Polymerisation des Knochenzements nach einem der Ansprüche 1 bis 8.

15. Formkörper nach Anspruch 14 zur Anwendung in einem Verfahren zur Behandlung und/oder Prävention von Infektionen, die durch Bakterien ausgelöst werden, als chirurgisches Implantat oder Teil eines Implantates, antibiotisches Implantat, Revisionsimplantat, Schraube, Nagel, chirurgische Platte, zur mechanischen Fixierung primärer Totalgelenkendoprothesen, zur mechanischen Fixierung von Revisions-Totalgelenkendoprothesen, zur Augmentation von osteoporotischem Knochengewebe und ganz besonders bevorzugt für die Vertebroplastie, Kyphoplastie und die Augmentation von Bohrlöchern in osteoporotischem Knochengewebe, zur Auffüllung von Knochenkavitäten, zur Femuroplastie, zur Herstellung von Spacern, zur mechanischen Fixierung von Gelenkprothesen, zur Abdeckung von Schädeldefekten oder zur Herstellung von Trägermaterialien für die lokale Antibiotika-Therapie oder als Trägermaterial für eine lokale Freisetzung von pharmazeutisch wirksamen Substanzen.

## Claims

1. Antibiotic polymerisable bone cement, comprising
(i) at least one monomer for radical polymerisation;
(ii) at least one organic polymer comprising at least one poly-methylmethacrylate and/or one poly-methylmethacrylate copolymer; and
(iii) at least one polymerisation initiator;
(iv) at least one radiopaquer;
**characterised in that**
the bone cement comprises
(v) as component **1,** daptomycin, a pharmacologically acceptable salt of daptomycin, a polymorphic form of daptomycin, a solvate and/or a hydrate containing daptomycin, **characterised in that**
the bone cement further comprises
(vi) a combination containing
a) as component **2,** a water-soluble calcium salt with a solubility in water at room temperature of more than/equal to 5 g/l; and
b) as component **3,** a poorly water-soluble calcium salt with a solubility in water at room temperature of less than 5 g/l.

2. Bone cement according to claim 1, **characterised in that**
(v) component **1,** daptomycin, a pharmacologically acceptable salt of daptomycin, a polymorphic form of daptomycin, a solvate and/or a hydrate containing daptomycin, and (vi) a) component **2,** the water-soluble calcium salt with a solubility in water at room temperature of more than/equal to 5 g/l as well as b) component **3,** the poorly water-soluble calcium salt with a solubility in water at room temperature of less than 5 g/l, each are present independent of each other in the form of particles of components **1, 2** or **3** or each are present independently as a particulate formulation containing, each independently, at least one of the components **1, 2** and/or **3** and at least one pharmacological excipient.

3. Bone cement according to any one of the claims 1 or 2, **characterised in that** at least component A comprises,
as (a4), at least one of the components **1, 2** and **3,** which each are present independent of each other in the form of particles of components **1, 2** or **3** or each are present independently as a particulate formulation containing, each independently, at least one of the components **1, 2** and/or **3,**
whereby component B is present in the form of a paste and comprises,
as (b4), at least one of the components 1, 2 and 3, which each are present independent of each other in the form of particles of components **1, 2** or **3** or each are present independently as a particulate formulation containing, each independently, at least one of the components **1, 2** and/or **3.**

4. Bone cement according to any one of the claims 1 to 3, **characterised in that**
a) the bone cement is present as (a4) and/or (b4) as particles of a combination of at least components **1** and **2** or, each independently, as a particulate formulation containing at least a combination of components **1** and **2** and at least one pharmacological excipient, or
b) the bone cement is present as (a4) and/or (b4) as particles of a combination of components **1, 2** and **3** or, each independently, as a particulate formulation containing the combination of components **1, 2** and **3** and at least one pharmacological excipient.

5. Bone cement according to any one of the claims 1 to 4, **characterised in that** at least one of the components **1, 2** and/or **3** comprises a mean particle size ranging from 1 to 250 µm.

6. Bone cement according to any one of the claims 1 to 5, **characterised in that**
a) component **2,** the water-soluble calcium salt with a solubility in water at room temperature of more than/equal to 5 g/l, comprises calcium gluconate, calcium glucuronate, calcium lactate, calcium acetate and/or calcium sorbate or a mixture containing at least two of said calcium salts.

7. Bone cement according to any one of the claims 1 to 6, **characterised in that** b) component **3,** the poorly water-soluble calcium salt with a solubility in water at room temperature of less than 5 g/l, comprises calcium sulfate dihydrate, calcium sulfate hemihydrate, alpha-tricalcium phosphate and/or beta-tricalcium phosphate.

8. Bone cement according to any one of the claims 1 to 7, **characterised in that** the bone cement contains at least a second antibiotic selected from the group of the aminoglycoside antibiotics and/or lincosamide antibiotics and/or ansamycin antibiotics and/or fluoroquinolone antibiotics and/or beta-lactam antibiotics.

9. Bone cement kit for the production of a bone cement according to any one of the claims 1 to 8, containing
two components A and B, whereby
component A is present as a paste and comprises
(a1) at least one monomer for radical polymerisation;
(a2) at least one organic polymer comprising at least one poly-methylmethacrylate and/or one poly-methylmethacrylate copolymer; and
(a3) at least one polymerisation initiator; and
component B is present as a paste and comprises
(b1) at least one monomer for radical polymerisation;
(b2) at least one organic polymer comprising at least one poly-methylmethacrylate and/or one poly-methylmethacrylate copolymer; and
(b3) at least one polymerisation promoter, **characterised in that** the kit comprises:
as component **1,** daptomycin, a pharmacologically acceptable salt of daptomycin, a polymorphic form of daptomycin, a solvate and/or a hydrate containing daptomycin, and
a combination containing
a) as component **2,** a water-soluble calcium salt with a solubility in water at room temperature of more than/equal to 5 g/l; and
b) as component **3,** a poorly water-soluble calcium salt with a solubility in water at room temperature of less than 5 g/l, whereby
component A comprises,
as (a4), at least one of the components **1, 2** and **3,** whereby component **1** is selected from daptomycin, a pharmacologically acceptable salt of daptomycin, a solvate and/or a hydrate containing daptomycin, component **2** is selected from a water-soluble calcium salt with a solubility in water at room temperature of more than/equal to 5 g/l, and component **3** is selected from a poorly water-soluble calcium salt with a solubility in water at room temperature of less than 5 g/l,
and
component B comprises,
as (b4), at least one of the components **1, 2** and **3,** whereby component **1** is selected from daptomycin, a pharmacologically acceptable salt of daptomycin, a polymorphic form of daptomycin, a solvate and/or a hydrate containing daptomycin, component **2** is selected from a water-soluble calcium salt with a solubility in water at room temperature of more than/equal to 5 g/l, and component **3** is selected from a poorly water-soluble calcium salt with a solubility in water at room temperature of less than 5 g/l.

10. Bone cement kit for the production of a bone cement according to any one of the claims 1 to 8, containing
two components A and B, whereby
component A is present as a powder and comprises
(a1) at least one powdered polymer comprising at least one polymethylmethacrylate and/or a powdered mixture comprising polymethylmethacrylate copolymers;
(a2) at least one powdered radiopaquer; and
(a3) at least one polymerisation initiator; and
component B is present as a liquid or paste and comprises
(b1) at least one monomer for radical polymerisation;
(b2) optionally, at least one organic polymer comprising at least one polymethylmethacrylate and/or one polymethylmethacrylate copolymer; and
(b3) at least one polymerisation promoter; **characterised in that** the kit comprises,
as component **1,** daptomycin, a pharmacologically acceptable salt of daptomycin, a polymorphic form of daptomycin, a solvate and/or a hydrate containing daptomycin, and
a combination containing
a) as component **2,** a water-soluble calcium salt with a solubility in water at room temperature of more than/equal to 5 g/l; and
b) as component **3,** a poorly water-soluble calcium salt with a solubility in water at room temperature of less than 5 g/l, whereby
at least component A comprises,
as (a4), at least one of the components **1, 2** and **3** selected from component **1,** daptomycin, a pharmacologically acceptable salt of daptomycin, a solvate and/or a hydrate containing daptomycin, component **2,** a water-soluble calcium salt with a solubility in water at room temperature of more than/equal to 5 g/l, and component **3,** a poorly water-soluble calcium salt with a solubility in water at room temperature of less than 5 g/l, and/or
whereby component B is present as a paste and comprises,
as (b4), at least one of the components **1, 2** and **3,** selected from component **1,** daptomycin, a pharmacologically acceptable salt of daptomycin, a polymorphic form of daptomycin, a solvate and/or a hydrate containing daptomycin, component **2,** a water-soluble calcium salt with a solubility in water at room temperature of more than/equal to 5 g/l, and component **3,** a poorly water-soluble calcium salt with a solubility in water at room temperature of less than 5 g/l.

11. Method for the production of a curable bone cement or a drug carrier for local release according to any one of the claims 1 to 8, by mixing two components A and B, whereby
component A is present as a paste and comprises
(a1) at least one monomer for radical polymerisation;
(a2) at least one organic polymer comprising at least one polymethylmethacrylate and/or one polymethylmethacrylate copolymer; and
(a3) at least one polymerisation promoter; and
component B is present as a paste and comprises
(b1) at least one monomer for radical polymerisation;
(b2) at least one organic polymer comprising at least one polymethylmethacrylate and/or one polymethylmethacrylate copolymer; and
(b3) at least one polymerisation promoter, **characterised in that**
as component **1,** daptomycin, a pharmacologically acceptable salt of daptomycin, a polymorphic form of daptomycin, a solvate and/or a hydrate containing daptomycin, and
a combination containing
a) as component **2,** a water-soluble calcium salt with a solubility in water at room temperature of more than/equal to 5 g/l; and
b) as component **3,** a poorly water-soluble calcium salt with a solubility in water at room temperature of less than 5 g/l, are being admixed, whereby component A comprises,
as (a4), at least one of the components **1, 2** and 3, whereby component **1** is selected from daptomycin, a pharmacologically acceptable salt of daptomycin, a solvate and/or a hydrate containing daptomycin, component **2** is selected from a water-soluble calcium salt with a solubility in water at room temperature of more than/equal to 5 g/l, and component **3** is selected from a poorly water-soluble calcium salt with a solubility in water at room temperature of less than 5 g/l, and
component B comprises,
as (b4), at least one of the components **1, 2** and **3,** whereby component **1** is selected from daptomycin, a pharmacologically acceptable salt of daptomycin, a polymorphic form of daptomycin, a solvate and/or a hydrate containing daptomycin, component **2** is selected from a water-soluble calcium salt with a solubility in water at room temperature of more than/equal to 5 g/l, and component **3** is selected from a poorly water-soluble calcium salt with a solubility in water at room temperature of less than 5 g/l.

12. Method for the production of a curable bone cement or a drug carrier for local release according to any one of the claims 1 to 8, by mixing two components A and B, whereby
component A is present as a powder and comprises
(a1) at least one powdered polymer comprising at least one polymethylmethacrylate and/or a powdered mixture comprising polymethylmethacrylate copolymers;
(a2) at least one powdered radiopaquer; and
(a3) at least one polymerisation initiator; and
component B is present as a liquid or paste and comprises
(b1) at least one monomer for radical polymerisation;
(b2) optionally, at least one organic polymer comprising at least one polymethylmethacrylate and/or one polymethylmethacrylate copolymer; and
(b3) at least one polymerisation promoter; **characterised in that**,
as component **1,** daptomycin, a pharmacologically acceptable salt of daptomycin, a polymorphic form of daptomycin, a solvate and/or a hydrate containing daptomycin, and
a combination containing
a) as component **2,** a water-soluble calcium salt with a solubility in water at room temperature of more than/equal to 5 g/l; and
b) as component **3,** a poorly water-soluble calcium salt with a solubility in water at room temperature of less than 5 g/l are being admixed, whereby at least component A comprises,
as (a4), at least one of the components **1, 2** and **3** selected from component **1,** daptomycin, a pharmacologically acceptable salt of daptomycin, a solvate and/or a hydrate containing daptomycin, component **2,** a water-soluble calcium salt with a solubility in water at room temperature of more than/equal to 5 g/l, and component **3,** a poorly water-soluble calcium salt with a solubility in water at room temperature of less than 5 g/l, and/or
whereby component B is present as a paste and comprises,
as (b4), at least one of the components **1, 2** and **3** selected from component **1,** daptomycin, a pharmacologically acceptable salt of daptomycin, a polymorphic form of daptomycin, a solvate and/or a hydrate containing daptomycin, component **2,** a water-soluble calcium salt with a solubility in water at room temperature of more than/equal to 5 g/l, and component **3,** a poorly water-soluble calcium salt with a solubility in water at room temperature of less than 5 g/l.

13. Drug carrier for local release obtainable according to a method according to either one of the claims 11 or 12.

14. Moulded body obtainable by moulding and polymerising the bone cement according to any one of the claims 1 to 8.

15. Moulded body according to claim 14 for use in a method for treatment and/or prevention of infections elicited by bacteria, as a surgical implant or part of an implant, antibiotic implant, revision implant, screw, nail, surgical plate, for mechanical fixation of primary total articular endoprostheses, for mechanical fixation of revision total articular endoprostheses, for augmentation of osteoporotic bone tissue and, particularly preferably, for vertebroplasty, kyphoplasty, and augmentation of drilling holes in osteoporotic bone tissue, for filling of bone cavities, for femuroplasty, for production of spacers, for mechanical fixation of articular prostheses, for coverage of skull defects or for production of carrier materials for local antibiotics therapy or as carrier material for local release of pharmaceutically effective substances.

## Revendications

1. Ciment osseux polymérisable antibiotique comprenant
(i) au moins un monomère polymérisable par voie radicalaire,
(ii) au moins un polymère organique comprenant au moins un polyméthylméthacrylate et/ou un copolymère de polyméthylméthacrylate, et
(iii) au moins un initiateur de polymérisation,
(iv) au moins un opacifiant radiographique,
**caractérisé en ce que**
le ciment osseux comprend
(v) en tant que composant 1 de la daptomycine, un sel pharmacologiquement compatible de daptomycine, une forme polymorphe de la daptomycine, un solvate et/ou un hydrate contenant de la daptomycine, **caractérisé en ce que** le ciment osseux comprend en outre
(vi) une combinaison contenant
a) en tant que composant 2 un sel de calcium soluble dans l'eau avec une solubilité dans l'eau à température ambiante supérieure ou égale à 5 g/l, et
b) en tant que composant 3 un sel de calcium faiblement soluble dans l'eau avec une solubilité dans l'eau à température ambiante inférieure à 5 g/l.

2. Ciment osseux selon la revendication 1, **caractérisé en ce que**
(v) le composant 1 la daptomycine, un sel pharmacologiquement compatible de daptomycine, une forme polymorphe de la daptomycine, un solvate et/ou un hydrate contenant de la daptomycine, et (vi) a) le composant 2 le sel de calcium soluble dans l'eau avec une solubilité dans l'eau à température ambiante supérieure ou égale à 5 g/l, ainsi que b) le composant 3 le sel de calcium faiblement soluble dans l'eau avec une solubilité dans l'eau à température ambiante inférieure à 5 g/l, sont présents à chaque fois indépendamment sous forme de particules des composants 1, 2 ou 3 ou sont présents à chaque fois indépendamment en tant qu'une formulation particulaire contenant à chaque fois indépendamment au moins un des composants 1, 2 et/ou 3 et au moins un adjuvant pharmacologique.

3. Ciment osseux selon une des revendications 1 ou 2, **caractérisé en ce qu'**au moins le composant A
comprend en tant que (a4) au moins un des composants 1, 2 et 3 qui est présent à chaque fois indépendamment sous forme de particules des composants 1, 2 ou 3 ou à chaque fois indépendamment en tant qu'une formulation particulaire contenant à chaque fois indépendamment au moins un des composants 1, 2 et/ou 3,
dans lequel le composant B est présent en tant que pâte et
comprend en tant que (b4) au moins un des composants 1, 2 et 3 qui est présent à chaque fois indépendamment sous forme de particules des composants 1, 2 ou 3 ou à chaque fois indépendamment en tant qu'une formulation particulaire contenant à chaque fois indépendamment au moins un des composants 1, 2 et/ou 3.

4. Ciment osseux selon une des revendications 1 à 3, **caractérisé en ce que**
a) le ciment osseux est présent en tant que (a4) et/ou (b4) en tant que particules d'une combinaison d'au moins les composants 1 et 2 ou à chaque fois indépendamment en tant qu'une formulation particulaire contenant au moins une combinaison des composants 1 et 2 et au moins un adjuvant pharmacologique, ou
b) le ciment osseux est présent en tant que (a4) et/ou (b4) en tant que particules d'une combinaison des composants 1, 2 et 3 ou à chaque fois indépendamment en tant qu'une formulation particulaire contenant la combinaison des composants 1, 2 et 3 et au moins un adjuvant pharmacologique.

5. Ciment osseux selon une des revendications 1 à 4, **caractérisé en ce que** au moins un des composants 1, 2 et/ou 3 présente une taille particulaire moyenne de 1 à 250 µm.

6. Ciment osseux selon une des revendications 1 à 5, **caractérisé en ce que**
a) le composant 2, le sel de calcium soluble dans l'eau avec une solubilité dans l'eau à température ambiante supérieure ou égale à 5 g/l comprend le gluconate de calcium, le glucuronate de calcium, le lactate de calcium, l'acétate de calcium et/ou le sorbate de calcium ou un mélange contenant au moins deux desdits sels de calcium.

7. Ciment osseux selon une des revendications 1 à 6, **caractérisé en ce que** b) le composant 3 le sel de calcium faiblement soluble dans l'eau avec une solubilité dans l'eau à température ambiante inférieure à 5 g/l comprend le sulfate de calcium dihydraté, l'hémihydrate de sulfate de calcium, l'alpha-phosphate tricalcique et/ou le bêta-phosphate tricalcique.

8. Ciment osseux selon une des revendications 1 à 7, **caractérisé en ce que** le ciment osseux contient au moins un second antibiotique sélectionné parmi le groupe des antibiotiques d'aminoglycoside et/ou des antibiotiques de lincosamide et/ou des antibiotiques d'ansamycine et/ou des antibiotiques de fluoroquinolone et/ou des antibiotiques de β-lactame.

9. Kit de ciment osseux pour la fabrication d'un ciment osseux selon une des revendications 1 à 8, contenant
deux composants A et B, dans lequel
le composant A est présent en tant que pâte et comprend
(a1) au moins un monomère polymérisable par voie radicalaire,
(a2) au moins un polymère organique comprenant au moins un polyméthylméthacrylate et/ou un copolymère de polyméthylméthacrylate, et
(a3) au moins un initiateur de polymérisation ; et
le composant B est présent en tant que pâte et comprend
(b1) au moins un monomère polymérisable par voie radicalaire,
(b2) au moins un polymère organique comprenant au moins un polyméthylméthacrylate et/ou un copolymère de polyméthylméthacrylate, et
(b3) au moins un accélérateur de polymérisation, **caractérisé en ce que** le kit comprend :
en tant que composant 1 de la daptomycine, un sel pharmacologiquement compatible de daptomycine, une forme polymorphe de la daptomycine, un solvate et/ou un hydrate contenant de la daptomycine, et
une combinaison contenant
a) en tant que composant 2 un sel de calcium soluble dans l'eau avec une solubilité dans l'eau à température ambiante supérieure ou égale à 5 g/l, et
b) en tant que composant 3 un sel de calcium faiblement soluble dans l'eau avec une solubilité dans l'eau à température ambiante inférieure à 5 g/l, dans lequel
le composant A
comprend en tant que (a4) au moins un des composants 1, 2 et 3, dans lequel le composant 1 est sélectionné parmi la daptomycine, un sel pharmacologiquement compatible de daptomycine, un solvate et/ou un hydrate contenant de la daptomycine, le composant 2 est sélectionné parmi un sel de calcium soluble dans l'eau avec une solubilité dans l'eau à température ambiante supérieure ou égale à 5 g/l, et le composant 3 est sélectionné parmi un sel de calcium faiblement soluble dans l'eau avec une solubilité dans l'eau à température ambiante inférieure à 5 g/l, et
le composant B
comprend en tant que (b4) au moins un des composants 1, 2 et 3, dans lequel le composant 1 est sélectionné parmi la daptomycine, un sel pharmacologiquement compatible de daptomycine, une forme polymorphe de la daptomycine, un solvate et/ou un hydrate contenant de la daptomycine, le composant 2 est sélectionné parmi un sel de calcium soluble dans l'eau avec une solubilité dans l'eau à température ambiante supérieure ou égale à 5 g/l, et le composant 3 est sélectionné parmi un sel de calcium faiblement soluble dans l'eau avec une solubilité dans l'eau à température ambiante inférieure à 5 g/l.

10. Kit de ciment osseux pour la fabrication d'un ciment osseux selon une des revendications 1 à 8, contenant
deux composants A et B, dans lequel
le composant A est présent en tant que poudre et comprend
(a1) au moins un polymère pulvérulent comprenant au moins un polyméthylméthacrylate et/ou un mélange pulvérulent comprenant des copolymères de polyméthylméthacrylate,
(a2) au moins un opacifiant radiographique pulvérulent, et
(a3) au moins un initiateur de polymérisation ; et
le composant B est présent en tant que fluide ou pâte et comprend
(b1) au moins un monomère polymérisable par voie radicalaire,
(b2) en option au moins un polymère organique comprenant au moins un polyméthylméthacrylate et/ou un copolymère de polyméthylméthacrylate, et
(b3) au moins un accélérateur de polymérisation, **caractérisé en ce que** le kit comprend :
en tant que composant 1 de la daptomycine, un sel pharmacologiquement compatible de daptomycine, une forme polymorphe de la daptomycine, un solvate et/ou un hydrate contenant de la daptomycine, et
une combinaison contenant
a) en tant que composant 2 un sel de calcium soluble dans l'eau avec une solubilité dans l'eau à température ambiante supérieure ou égale à 5 g/l, et
b) en tant que composant 3 un sel de calcium faiblement soluble dans l'eau avec une solubilité dans l'eau à température ambiante inférieure à 5 g/l, dans lequel
au moins le composant A
comprend en tant que (a4) au moins un des composants 1, 2 et 3 qui est sélectionné parmi le composant 1 la daptomycine, un sel pharmacologiquement compatible de daptomycine, un solvate et/ou un hydrate contenant de la daptomycine, le composant 2 un sel de calcium soluble dans l'eau avec une solubilité dans l'eau à température ambiante supérieure ou égale à 5 g/l, et le composant 3 un sel de calcium faiblement soluble dans l'eau avec une solubilité dans l'eau à température ambiante inférieure à 5 g/l, et/ou
dans lequel le composant B est présent en tant que pâte et
comprend en tant que (b4) au moins un des composants 1, 2 et 3 qui est sélectionné parmi le composant 1 la daptomycine, un sel pharmacologiquement compatible de daptomycine, une forme polymorphe de la daptomycine, un solvate et/ou un hydrate contenant de la daptomycine, le composant 2 un sel de calcium soluble dans l'eau avec une solubilité dans l'eau à température ambiante supérieure ou égale à 5 g/l, et le composant 3 un sel de calcium faiblement soluble dans l'eau avec une solubilité dans l'eau à température ambiante inférieure à 5 g/l.

11. Procédé de fabrication d'un ciment osseux durcissable ou d'un support d'ingrédient actif à libération locale selon une des revendications 1 à 8, en ce que deux composants A et B sont mélangés, dans lequel
le composant A est présent en tant que pâte et comprend
(a1) au moins un monomère polymérisable par voie radicalaire,
(a2) au moins un polymère organique comprenant au moins un polyméthylméthacrylate et/ou un copolymère de polyméthylméthacrylate, et
(a3) au moins un initiateur de polymérisation ; et
le composant B est présent en tant que pâte et comprend
(b1) au moins un monomère polymérisable par voie radicalaire,
(b2) au moins un polymère organique comprenant au moins un polyméthylméthacrylate et/ou un copolymère de polyméthylméthacrylate, et
(b3) au moins un accélérateur de polymérisation, **caractérisé en ce que** en tant que composant 1 de la daptomycine, un sel pharmacologiquement compatible de daptomycine, une forme polymorphe de la daptomycine, un solvate et/ou un hydrate contenant de la daptomycine, et
une combinaison contenant
a) en tant que composant 2 un sel de calcium soluble dans l'eau avec une solubilité dans l'eau à température ambiante supérieure ou égale à 5 g/l, et
b) en tant que composant 3 un sel de calcium faiblement soluble dans l'eau avec une solubilité dans l'eau à température ambiante inférieure à 5 g/l, sont mélangés, dans lequel
le composant A
comprend en tant que (a4) au moins un des composants 1, 2 et 3, dans lequel le composant 1 est sélectionné parmi la daptomycine, un sel pharmacologiquement compatible de daptomycine, un solvate et/ou un hydrate contenant de la daptomycine, le composant 2 est sélectionné parmi un sel de calcium soluble dans l'eau avec une solubilité dans l'eau à température ambiante supérieure ou égale à 5 g/l, et le composant 3 est sélectionné parmi un sel de calcium faiblement soluble dans l'eau avec une solubilité dans l'eau à température ambiante inférieure à 5 g/l, et
le composant B
comprend en tant que (b4) au moins un des composants 1, 2 et 3, dans lequel le composant 1 est sélectionné parmi la daptomycine, un sel pharmacologiquement compatible de daptomycine, une forme polymorphe de la daptomycine, un solvate et/ou un hydrate contenant de la daptomycine, le composant 2 est sélectionné parmi un sel de calcium soluble dans l'eau avec une solubilité dans l'eau à température ambiante supérieure ou égale à 5 g/l, et le composant 3 est sélectionné parmi un sel de calcium faiblement soluble dans l'eau avec une solubilité dans l'eau à température ambiante inférieure à 5 g/l.

12. Procédé de fabrication d'un ciment osseux durcissable ou d'un support d'ingrédient actif à libération locale selon une des revendications 1 à 8, en ce que deux composants A et B sont mélangés, dans lequel le composant A est présent en tant que poudre et comprend
(a1) au moins un polymère pulvérulent comprenant au moins un polyméthylméthacrylate et/ou un mélange pulvérulent comprenant des copolymères de polyméthylméthacrylate,
(a2) au moins un opacifiant radiographique pulvérulent, et
(a3) au moins un initiateur de polymérisation ; et
le composant B est présent en tant que fluide ou pâte et comprend
(b1) au moins un monomère polymérisable par voie radicalaire,
(b2) en option au moins un polymère organique comprenant au moins un polyméthylméthacrylate et/ou un copolymère de polyméthylméthacrylate, et
(b3) au moins un accélérateur de polymérisation, **caractérisé en ce que** en tant que composant 1 de la daptomycine, un sel pharmacologiquement compatible de daptomycine, une forme polymorphe de la daptomycine, un solvate et/ou un hydrate contenant de la daptomycine, et
une combinaison contenant
a) en tant que composant 2 un sel de calcium soluble dans l'eau avec une solubilité dans l'eau à température ambiante supérieure ou égale à 5 g/l, et
b) en tant que composant 3 un sel de calcium faiblement soluble dans l'eau avec une solubilité dans l'eau à température ambiante inférieure à 5 g/l, sont mélangés,
dans lequel au moins le composant A
comprend en tant que (a4) au moins un des composants 1, 2 et 3 qui est sélectionné parmi le composant 1 la daptomycine, un sel pharmacologiquement compatible de daptomycine, un solvate et/ou un hydrate contenant de la daptomycine, le composant 2 un sel de calcium soluble dans l'eau avec une solubilité dans l'eau à température ambiante supérieure ou égale à 5 g/l, et le composant 3 un sel de calcium faiblement soluble dans l'eau avec une solubilité dans l'eau à température ambiante inférieure à 5 g/l, et/ou
dans lequel le composant B est présent en tant que pâte et
comprend en tant que (b4) au moins un des composants 1, 2 et 3 qui est sélectionné parmi le composant 1 la daptomycine, un sel pharmacologiquement compatible de daptomycine, une forme polymorphe de la daptomycine, un solvate et/ou un hydrate contenant de la daptomycine, le composant 2 un sel de calcium soluble dans l'eau avec une solubilité dans l'eau à température ambiante supérieure ou égale à 5 g/l, et le composant 3 un sel de calcium faiblement soluble dans l'eau avec une solubilité dans l'eau à température ambiante inférieure à 5 g/l.

13. Support d'ingrédient actif à libération locale pouvant être obtenu selon un procédé selon une des revendications 11 ou 12.

14. Corps moulé pouvant être obtenu par moulage et polymérisation du ciment osseux selon une des revendications 1 à 8.

15. Corps moulé selon la revendication 14 pour l'application dans un procédé de traitement et/ou prévention d'infections qui sont déclenchées par des bactéries, en tant qu'implant chirurgical ou partie d'un implant, implant antibiotique, implant de révision, vis, clou, plaque chirurgicale, pour la fixation mécanique d'endoprothèses articulaires totales primaires, pour la fixation mécanique d'endoprothèses articulaires totales de révision, pour l'augmentation de tissu osseux ostéoporotique et de manière tout particulièrement préférée pour la vertébroplastie, la kyphoplastie et l'augmentation de trous de trépan dans du tissu osseux ostéoporotique, pour le remplissage de cavités osseuses, pour la fémoroplastie, pour la fabrication d'écarteurs, pour la fixation mécanique de prothèses articulaires, pour le recouvrement de défauts crâniens ou pour la fabrication de matériaux de support pour l'antibiothérapie locale ou en tant que matériau de support pour une libération locale de substances pharmaceutiquement actives.
